# EUROPEAN PATENT SPECIFICATION

(11) **EP 3 582 693 B1**
(45) Date of publication and mention of the grant of the patent: **07.04.2021**
(21) Application number: 18710765.1
(22) Date of filing: 14.02.2018
(51) Int. Cl.: A61B 8/08, A61B 8/00, A61B 17/34

(54) **FOCUS TRACKING IN ULTRASOUND SYSTEM FOR DEVICE TRACKING**
FOKUSVERFOLGUNG IN ULTRASCHALLSYSTEM ZUR VORRICHTUNGSVERFOLGUNG
SUIVI DE MISE AU POINT DANS UN SYSTÈME À ULTRASONS POUR SUIVI DE DISPOSITIF

(30) Priority: 14.02.2017 US 201762458763 P
(43) Date of publication of application: 25.12.2019
(73) Proprietor: Koninklijke Philips N.V., 5656 AG Eindhoven (NL)
(72) Inventor: POLAND, Mckee, Dunn, 5656 AE Eindhoven (NL)
(74) Representative: Philips Intellectual Property & Standards
(86) International application number: PCT/EP2018/053714
(87) International publication number: WO 2018/149887

(56) References cited:
- US-A1- 2015 342 572
- US-B1- 6 629 929
- US-B2- 9 282 946

## Description

### BACKGROUND:

### Technical Field

This disclosure relates to ultrasound devices and more particularly to automatically selecting a transmit focal depth of an ultrasound probe to closely match and track a needle tip depth.

### Description of the Related Art

Precise visualization of objects such as needles or catheters and real-time localization with respect to imaged anatomy are needed for minimally invasive interventions. Intraoperative ultrasound is often used for these purposes. Various ultrasound systems are available in the market which utilize some method for tracking the location of an object in the body of the patient. Such systems share the common attribute that each detected position of the object is digitally represented in the system, allowing display of the positions, and that the positions are updated periodically, typically in conjunction with active scanning, so that the real time ultrasound image display can also show the detected location of the object being tracked. Some systems offer a means of showing the path of the detected object in the image, either as history (where the object came from), or future extrapolation (where it will go if moved in the same direction), or both. Generating such a projected path is typically by means of a method well understood in the art. One method is to include a mechanical fixture like a needle guide mounted on the ultrasound probe which simply constrains the object to follow a predetermined path, i.e., to physically constrain the path of the object with respect to the ultrasound probe as the object is inserted. Other means include locating the device such as by magnetic or electro-magnetic (EM) sensing of the location of the object with respect to similar sensing of the ultrasound probe position.

These systems suffer from complex, expensive parts and circuitry, susceptibility to interference, positional ambiguity due to the deformation of the object (such as bending of the needle), workflow burden such as the obligation to calibrate the positional sensing, etc. There is one system which requires no physical registration of the relative positions of the ultrasound probe (and thus the displayed image) and the object whose position is displayed in the image. U.S. Patent No. 9,282,946, describes a system wherein an acoustic signal from the probe is used to activate an acoustic sensor on the tracked object, and via the timing of a returned electrical signal from the object, detect the position of the object with respect to the image itself, thereby obviating all mechanical, magnetic, electromagnetic (EM), or other mechanisms for tracking, and thus also eliminating their cost, complexity, calibration, and susceptibility to error.

In any ultrasound imaging system that also tracks and displays the position of an object, it would be desirable to more clearly display the tracked object and its surrounding anatomy throughout the ongoing series of displayed images (i.e., through time) as the object is moved from the shallow depths in the body to deeper depths. The simplified, low cost system of U.S. Patent No. 9,282,946, which uses only an acoustic sensor on the object for position detection, allows most accurate and efficient tracking of an object when the transmit focus of the imaging is near the physical depth of the acoustic sensor. It is desirable to automatically maintain the accuracy of the tracking as well as the image quality of the anatomy in the vicinity of the object as the object is moved to deeper or shallower depths.

Document US 2015/0342572 A1 discloses a method and system for tracking a location of an object while the object is disposed within a region of interest within biological tissue, the location of the object being determined with respect to a tracking coordinate frame; generate acoustic images of the region of interest, the acoustic images being generated with respect to an acoustic image coordinate frame which is different from the tracking coordinate frame; transform the location of the object from the tracking coordinate frame to the acoustic image coordinate frame; and automatically adjust at least one image resolution parameter of the acoustic images in response to the location of the object with respect to the acoustic image coordinate frame.

As further background, a very brief review of ultrasound probes and imaging follows. The versatility of a diagnostic ultrasound system is largely determined by the types of probes which can be used with the system. Linear array transducer probes are generally preferred for abdominal and small parts imaging and phased array transducer probes are preferred for cardiac imaging. Probes may have 1D or 2D array transducers for two dimensional or three dimensional imaging. Indwelling probes are in common use, as are specialty probes such as surgical probes. Each type of probe can operate at a unique frequency range and have a unique aperture and array element count. Some ultrasound systems are designed for grayscale operation or operation at the transmit frequency such as for greyscale and color Doppler imaging while others can additionally perform harmonic imaging. For each of the intended imaging modes, the functional characteristics of the probes, such as physical aperture, transducer element spacing, passband frequencies, etc. determine the requirements for transmitting ultrasound pulses and processing the received echoes. The variation in probe characteristics and functionality means that the processing system operable with a variety of probes must be reprogrammed each time a different probe is put to use.

An example of an object that is tracked during an ultrasound procedure is a needle. During needle biopsy and some interventional therapy, clinicians insert a needle into a subject, such as the body, to reach a target region of interest. For regional anesthesia, a needle is used to deliver anesthetic to the vicinity of a target nerve bundle in the body, typically in preparation for a surgical procedure. Usually ultrasound imaging is used for live monitoring of the needle insertion procedure. To perform a safe and successful insertion, it is necessary to locate the needle accurately in the guided ultrasound image. Unfortunately, in clinical practice the visibility of the needle itself in the conventional ultrasound image is poor, resulting in difficulty for clinicians to insert the needle accurately. Hence the desirability of an accurate needle tracking system and further, a means to maintain optimal imaging characteristics in the vicinity of the needle tip depth.

Different techniques have been used to achieve better needle visualization in ultrasound images, for example, adaptively steering the ultrasound beam towards the needle to improve the acoustic reflection of the needle and compounding with the non-steered ultrasound image; manipulating the needle surface coating, geometry and diameter to enhance acoustic reflection; providing an extra optical, magnetic, or electro-magnetic position sensor on the needle to track the needle location in the ultrasound image, etc. In these techniques, either a specially designed needle is used, or an extra position sensor is attached to the needle, or the ultrasound imaging system is manipulated to enhance the visualization of the needle. Those approaches lead to an increase of the cost of providing enhanced needle visualization. In contrast, the simple system mentioned above, which utilizes only an acoustic sensor on the object to provide an electrical signal to the system for location detection, reduces the cost and complexity of the tracking apparatus while increasing its accuracy. Additionally, it presents an opportunity to automatically optimize both tracking accuracy and image quality in the vicinity of the tracked object.

### SUMMARY

In accordance with the present principles, an ultrasound system includes an ultrasound probe having a transducer array, an acquisition module coupled to the transducer array and a transceiver coupled to the acquisition module for communicating with an adjustment module, wherein the adjustment module is configured to automatically make adjustments to the beamformed acoustic pulse characteristics. The adjustments to the pulse characteristics are derived from pre-established user image adjustment selections available on a user interface.

A system includes at least one transducer configured for removable securement to a subject and a signal processor configured for removable connection to the at least one transducer, the signal processor configured to apply an electrical signal to the at least one transducer by communicating with an acquisition module, so as to cause the at least one transducer to deliver ultrasound energy to the site in the patient. The system further includes an image processor for generating ultrasound images from acoustic data received by the transducer, and for further identifying a distal end of a medical instrument within a region of interest and establishing a relationship between a depth of a distal end of the medical instrument and a focal depth displayed on a display.

A method for determining a depth of a medical device within a subject is disclosed in U.S. Patent No. 9,282,946. The method of the invention described herein further includes automatically making adjustments to beamformed acoustic pulse locations and deriving the adjustments to the pulse locations from pre-established user image adjustment selections available on a user interface.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing an ultrasonic diagnostic imaging system, in accordance with one embodiment;
FIG. 2 is a diagram showing a needle tip tracking (NTT) system in communication with an ultrasound system, in accordance with one embodiment;
FIG. 3 is a diagram showing an ultrasound image depicting a needle at a first focal depth, in accordance with one embodiment;
FIG. 4 is a diagram showing an ultrasound image depicting a needle at a second focal depth, in accordance with another embodiment;
FIG. 5 is a diagram showing different predetermined focal depths that the ultrasound systems supports and automatically selects from, in accordance with one embodiment; and
FIG. 6 is a flow diagram showing a method for automatically selecting a transmit focal depth of an ultrasound probe to closely match and track the needle tip depth, in accordance with illustrative embodiments.

### DETAILED DESCRIPTION OF EMBODIMENTS

The invention is defined by the claims.

In accordance with the present principles, systems, devices and methods are provided for automatically selecting a transmit focal depth of an ultrasound probe to closely match and track a needle tip depth. The present principles provide embodiments where the systems, devices and methods enable the transmit focal depth selection to be taken from a predetermined set of legal depths that the ultrasound system supports, that in fact can be reached by the user via user controls and that have been optimized for imaging. By selecting from the supported set of optimized transmit focal depths, the system maintains optimum imaging in the region of the needle tip and simultaneously optimizes the performance of the needle tip tracking.

It should be understood that the present invention will be described in terms of medical instruments; however, the teachings of the present invention are much broader and are applicable to any acoustic instruments. In some embodiments, the present principles are employed in tracking or analyzing instruments in complex biological or mechanical systems. In particular, the present principles are applicable to internal and/or external tracking procedures of biological systems and procedures in all areas of the body such as the lungs, gastro-intestinal tract, excretory organs, blood vessels, etc. The functional elements depicted in the FIGS. may be implemented in various combinations of hardware and software and provide functions which may be combined in a single element or multiple functional elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD.

Reference in the specification to "one embodiment" or "an embodiment" of the present principles, as well as other variations thereof, means that a particular feature, structure, characteristic, and so forth described in connection with the embodiment is included in at least one embodiment of the present principles. Thus, the appearances of the phrase "in one embodiment" or "in an embodiment", as well any other variations, appearing in various places throughout the specification are not necessarily all referring to the same embodiment.

It is to be appreciated that the use of any of the following "/", "and/or", and "at least one of', for example, in the cases of "A/B", "A and/or B" and "at least one of A and B", is intended to encompass the selection of the first listed option (A) only, or the selection of the second listed option (B) only, or the selection of both options (A and B). As a further example, in the cases of "A, B, and/or C" and "at least one of A, B, and C", such phrasing is intended to encompass the selection of the first listed option (A) only, or the selection of the second listed option (B) only, or the selection of the third listed option (C) only, or the selection of the first and the second listed options (A and B) only, or the selection of the first and third listed options (A and C) only, or the selection of the second and third listed options (B and C) only, or the selection of all three options (A and B and C). This may be extended, as readily apparent by one of ordinary skill in this and related arts, for as many items listed.

It will also be understood that when an element such as a layer, region or material is referred to as being "on" or "over" another element, it can be directly on the other element or intervening elements may also be present. In contrast, when an element is referred to as being "directly on" or "directly over" another element, there are no intervening elements present. It will also be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

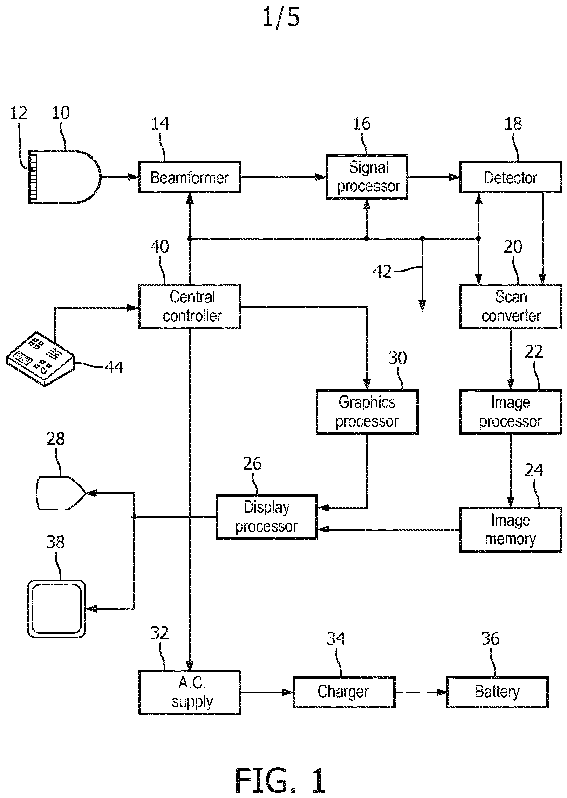

Referring now to the drawings in which like numerals represent the same or similar elements and initially to FIG. 1, an ultrasonic diagnostic imaging system is illustratively shown in accordance with one embodiment.

Referring first to FIG. 1, an ultrasonic diagnostic imaging system constructed in accordance with the principles of the present invention is shown in block diagram form. An ultrasound probe 10 transmits and receives ultrasound waves from the piezoelectric elements of an array of transducer elements 12. For imaging a planar region of the body a one-dimensional (1-D) array of elements may be used, and for imaging a volumetric region of the body a two-dimensional (2-D) array of elements may be used to steer and focus ultrasound beams over the image region. A transmit beamformer actuates elements of the array to transmit ultrasound waves into the subject. The signals produced in response to the reception of ultrasound waves are coupled to a receive beamformer 14. The beamformer 14 delays and combines the signals from the individual transducer elements to form coherent beamformed echo signals. When the probe includes a 2-D array for 3D imaging, it may also include a microbeamformer which does partial beamforming in the probe by combining signals from a related group ("patch") of transducer elements as described in U.S. Pat. No. 6,709,394. In that case the microbeamformed signals are coupled to the main beamformer 14 in the system which completes the beamforming process.

The beamformed echo signals are coupled to a signal processor 16 which processes the signals in accordance with the information desired. The signals may be filtered, for instance, and/or harmonic signals may be separated out for processing. The processed signals are coupled to a detector 18 which detects the information of interest. For B mode imaging amplitude detection is usually employed, whereas for spectral and color Doppler imaging the Doppler shift or frequency can be detected. The detected signals are coupled to a scan converter 20 where the signals are coordinated to the desired display format, e.g., in a Cartesian coordinate system. Common display formats used are sector, rectilinear, and parallelogram display formats. The scan converted signals are coupled to an image processor for further desired enhancement such as persistence processing. The scan converter may be bypassed for some image processing. For example the scan converter may be bypassed when 3D image data is volume rendered by the image processor by direct operation on a 3D data set. The resulting two dimensional or three dimensional image is stored temporarily in an image memory 24, from which it is coupled to a display processor 26. The display processor 26 produces the necessary drive signals to display the image on a docking station image display 28 or the flat panel display 38 of the portable system. The display processor also overlays the ultrasound image with graphical information from a graphics processor 30 such as system configuration and operating information, patient identification data, and the time and date of the acquisition of the image.

A central controller 40 responds to user input from the user interface and coordinates the operation of the various parts of the ultrasound system, as indicted by the arrows drawn from the central controller 40 to the beamformer 14, the signal processor 16, the detector 18, and the scan converter 20, and the arrow 42 indicating connections to the other parts of the system. The user control panel 44 is shown coupled to the central controller 40 by which the operator enters commands and settings for response by the central controller 40. The central controller 40 may also be coupled to an a.c. power supply 32 to cause the a.c. supply to power a battery charger 34 which charges the battery 36 of the portable ultrasound system when the portable system is docked in the docking station.

It is thus seen that, in this embodiment, the partitioning of the components of FIG. 1 is as follows. The central controller 40, beamformer 14, signal processor 16, detector 18, scan converter 20, image processor 22, image memory 24, display processor 26, graphics processor 30, flat panel display 38, and battery 36 reside in the portable ultrasound system. The control panel 44, display 28, a.c. supply 32 and charger 34 reside on the docking station. In other embodiments the partitioning of these subsystems may be done in other ways as design objectives dictate.

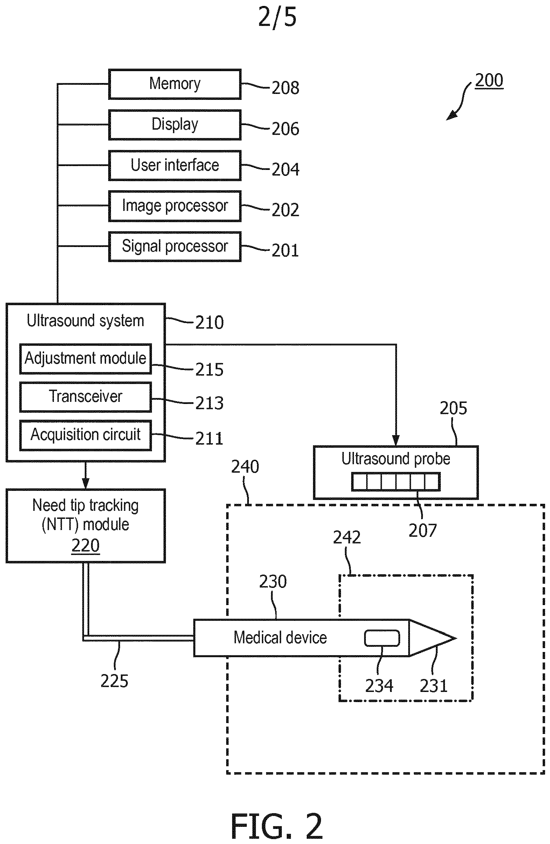

Referring to FIG. 2, a diagram showing a needle tip tracking (NTT) system in communication with an ultrasound system is presented in accordance with one embodiment.

The tracking system 200 includes an ultrasound system 210 in communication with a needle tip tracking (NTT) module 220, which is connected to medical device 230 preferably via a cable 225. The medical device 230 can be, e.g., a medical needle 230, but also a catheter or other device used in medical procedures, whose position it is beneficial to track. The ultrasound system 210 may include a signal processor 201, an image processor 202, a user interface 204, a display 206, and a memory 208. Additionally, an ultrasound probe 205 may be connected to the ultrasound system 210, the ultrasound probe 205 including a plurality of transducer elements 207. The ultrasound probe 205 may be positioned adjacent the subject 240. The subject 240 can be, e.g., a patient.

The ultrasound system 210 can further include an acquisition module 211, a transceiver 213, and an adjustment module 215 in communication with the ultrasound probe 205.

The acquisition module 211 provides communication between the microbeamformer 14 (FIG. 1) and the transceiver 213. The acquisition module 211 provides timing and control signals to the microbeamformer 14, directing the transmission of ultrasound waves and receiving at least partially beamformed echo signals from the microbeamformer 14, which are demodulated and detected (and optionally scan converted) and communicated to the transceiver 213.

The needle 230 is inserted into a volume or region of interest 242 of the subject 240. The distal end of the needle 230 may be, e.g., a pointed end or beveled tip 231. Of course, one skilled in the art may contemplate a number of different design configurations for the distal end of the needle 230. U.S. Patent No. 9,282,946, provides further information regarding the tracking system 200 and various beamforming techniques.

The needle is tracked, preferably from the point of entry at the skin surface all the way to the point where it stops insertion. For regional anesthesia, for instance, the stopping point is near a visualized nerve bundle, at which point the anesthetic is injected through the needle cannula so that it optimally bathes the nerve bundle.

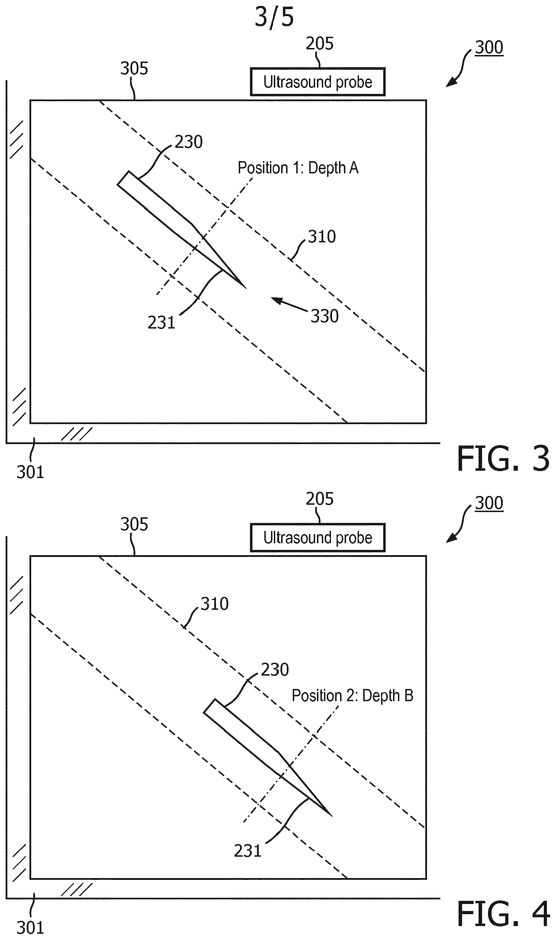

FIG. 3 is a diagram showing an ultrasound image depicting a needle at a first focal depth, in accordance with one embodiment, whereas FIG. 4 is a diagram showing an ultrasound image depicting a needle at a second focal depth, in accordance with the embodiment.

The diagram illustrates an ultrasound image 305. The ultrasound image 305 is shown on a screen 300 of a display device 301. The ultrasound image 305 depicts the needle 230 travelling along a tracked path 330 within, e.g., a lumen 310. The distal end of the needle 230 includes a pointed end or beveled tip 231.

The needle 230 is shown in a first position near transmit focal depth A in FIG. 3.

The needle 230 is shown in a second position near transmit focal depth B in FIG. 4.

Depths A and B can be referred to as transmit focal depths. The transmit focal depth is the depth at which each acoustic line's generated acoustic pulses are focused in the medium. The transmit focal depth is preferentially less than the imaging depth, and is selectable amongst a pre-determined set of depths whose acoustic power characteristics are carefully measured and limited in accordance with United States FDA regulation. It is the choice of the active transmit focal depth that is controlled by the system of the invention.

In operation, an acoustic sensor 234 on the tip 231 of the needle 230 is "seeing" the effect of the transmit focus only, and generating its instant electrical response to the scan lines that sweep across it with whatever transmit focal depth is active. The closer the transmit focal depth is to the actual depth of the acoustic sensor 234 on the needle 230, the more precise the detection of location can be because the signal-to-noise ratio of the needle's received pulse is higher when transmit focus from probe 205 is close to the depth of the needle sensor in the tissue. The specificity of the received signal is increased in that an acoustic signal detected by acoustic sensor 234 is both relatively higher in amplitude at the focal depth, and also higher in amplitude than the detected signals of adjacent lines in the sweep. Thus, both depth and lateral resolution of the detected signal are improved when the transmit focal depth is close to the needle tip depth. The exemplary embodiments of the present invention relate to automatically selecting the transmit focus or focal depth of the ultrasound probe 205 to closely match and track the needle tip depth.

Referring to FIGS. 2-4 in tandem, in an ultrasound system which includes an object location apparatus, wherein the object location apparatus utilizes the ultrasound acoustic pulses generated by the ultrasound system to energize the tracking sensor, a method of automatically making adjustments to the beamformed acoustic pulse locations is introduced in order to increase the strength and specificity of the received signal from the tracking sensor, thereby improving tracking accuracy while at the same time achieving higher image quality in the vicinity of the tracked object. The user is thus spared the effort of manually making the image adjustments while occupied with a medical procedure.

Moreover, in preferred embodiments, the adjustments to pulse locations are chosen solely from selections that the user could manually choose for the image adjustment. As a result, the ultrasound system requires no special acoustic power characterization for tracking, no special scan line patterns, and no altered image optimization. In essence, the ultrasound system is making optimal user interface choices automatically for both tracking and image quality.

In the needle tracking system that accompanies the ultrasound scanning system, the focus track mechanism automatically moves the transmit focus to the depth of the needle tip, thus allowing the physician to better resolve both the needle location and anatomic structures in the region of interest. As the needle moves deeper or shallower, the transmit focus follows it automatically. The system thereby optimizes both needle tracking and the view of the target structures that the needle is approaching.

The focus track mechanism uses the detected needle depth to generate increments or decrements to the transmit focus/focal depth. In a preferred embodiment, the system responds to the increment or decrement requests as it would for a focus up/down toggle in the user interface. In other words, it moves the focus shallower or deeper within the limits of the imaging mode, depth, and imaging preset. It may limit the focus movement at extreme shallow or deep depths due to lack of an available choice in the direction requested, but in such cases there is little to be gained from a focus depth change.

To generate an up/down request, the current detected needle tip depth is compared to the current transmit focus depth. This comparison is preferably done periodically, but it may be done continuously or as needed. The focus change requests are also periodic and executed as needed, i.e., if the needle depth has substantially changed. Hysteresis can be used to avoid oscillation in focus depth. For example, if the needle tip is past a minimum distance above or below the current focal depth, then the focal depth increment or decrement is requested. A typical period between focus depth updates is, e.g., 25 scan frames. A typical hysteresis depth is, e.g., 1 cm. These settings may depend on the probe model and imaging preset that is in use.

In summary, in the preferred embodiment, the transmit focus tracks the detected needle depth, snapping to one of the available transmit depth focus choices, that is, from the set that has been acoustically power tested, approved to be within acoustic power limits, and optimized for imaging. As a result of this action, the image is more finely resolved near the needle tip and the tip position is also more accurately tracked because it is insonicated by focused beams. Stated differently, the transmit focal depth selection is taken from a predetermined set of legal depths that the ultrasound system supports, that in fact can be reached by the user via user controls and that have been optimized for imaging. By selecting from the supported set of optimized transmit focal depths, the ultrasound system maintains optimum imaging in the region of the needle tip and simultaneously optimizes the performance of needle tip tracking.

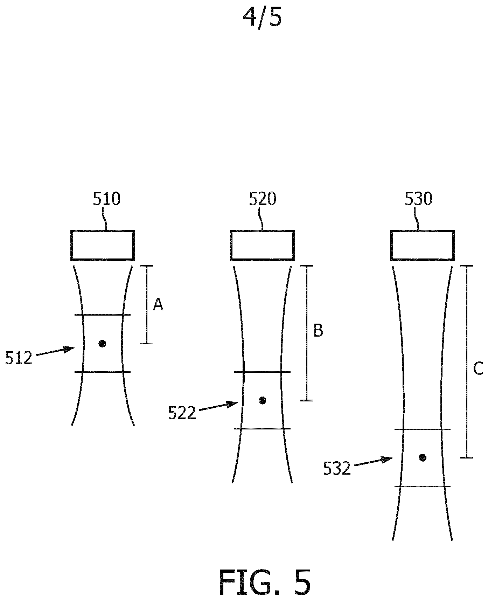

FIG. 5 is a diagram showing different predetermined focal depths that the ultrasound systems supports and automatically selects from, in accordance with one embodiment.

Transducers are designed with, typically, a fixed beam focus profile in the short (elevation) axis as determined by an acoustic lens, and the ability to control the focus depth in the long (lateral) axis by means of time phasing of pulse transmission and reception on acoustic elements of the transducer sensor. The system thus can control the focal depth of transmitted beams through transmit beamforming of the acoustic elements, as is well understood in the art, by controlling the activation of transmit pulses from the individual sensor elements. The focal depth may be determined by the time delay between the electrical pulses. This can be changed electronically to focus pulses to give good image detail at various depths within the body rather than just one depth as with the fixed focus transducer.

In a first example, an ultrasound probe 510 can emit beams toward a first focal zone 512. In a second example, an ultrasound probe 520 can emit beams toward a second focal zone 522. In a third example, an ultrasound probe 530 can emit beams toward a third focal zone 532. The first focal zone 512 can have a depth A, the second focal 522 zone can have a depth B, and the third focal zone 532 can have a depth C. Depth C is greater than depth B and depth A. Depth B is greater than depth A. Referring again to FIG. 5, the outer curved lines for each focal zone represent a contour of equal acoustic power, narrowing in an hourglass shape to the nominal focal depth which is represented by the dot at the nominal focal depth. This is a typical transmit focus profile well understood in the art to be the result of phased array beamforming. A variety of techniques are typically employed to optimize the shape of the contour, its width, etc., by adjusting the transmit aperture (i.e., the number of acoustic elements that are active in the array), the characteristics of the transmit pulse, etc. All such parameters are optimized and fixed for a given focal depth so that the acoustic power may then be characterized, as mentioned above, for each chosen focal zone. By choosing from the pre-determined set of focal depths, the system also chooses the accompanying beamforming, aperture, and pulse characteristics. For the purposes of discussion herein, when we refer to a focal depth, it represents the setting of all the associated, fixed transmit beam characteristics of that focal depth.

For each scan line in a sweep, the beam steering is varied, again by a different setting of electrical transmit pulse delays, as is well understood in the art. For each of the scan lines, the focal depth of the transmit beams is typically and preferably equal to that of the other scan lines, according to the choice of focal depth active in the system. Thus, all the scan lines of a sweep share the same focal depth, but vary in direction.

Therefore, the transmit beams shown in FIG. 5 are for convenience shown with centered steering, but are taken to be typical for any scan line steering angle. The distance between the ultrasound probes 510, 520, 530 and the focus zones 512, 522, 532 can be designated as the focal depth. The transmit focal depths A, B, C may be predetermined or predefined depths whose acoustic power characteristics are carefully measured and limited in accordance with FDA regulation. The choice of such active and predetermined or predefined or pre-established transmit focal depths is controlled by the exemplary embodiments of the present invention. In other words, one of these predetermined transmit focus depths is automatically chosen by the ultrasound system (without user intervention) to closely match and track the needle tip depth. Thus, transmit focal depths are automatically taken or selected from a set of legal or appropriate or pre-established depths that the ultrasound system supports.

As an addition or alternative to adjusting the transmit focus as described, any of many possible alterations to the acoustic transmit pulse characteristics may be performed in accordance with the detected object depth. For example, the acoustic pulse frequency, pulse length (number of transmit cycles), scan line density (number of scan lines in the sweep of the scan frame), etc. may also be optimally varied if they provide benefit to imaging or object tracking. However, in accordance with the principles of the invention, only pre-defined variations in the acoustic pulse characteristics which can be actuated by means of controls on a user interface should be considered, since only those will typically meet the requirement that they have been verified for legal acoustic power output and optimized for display. Collectively, then, the invention may generalize from adjustment of just transmit focal depth to in fact include any of a pre-defined set of acoustic transmit pulse characteristics, including focal depth, as potential adjustments according to detected object depth.

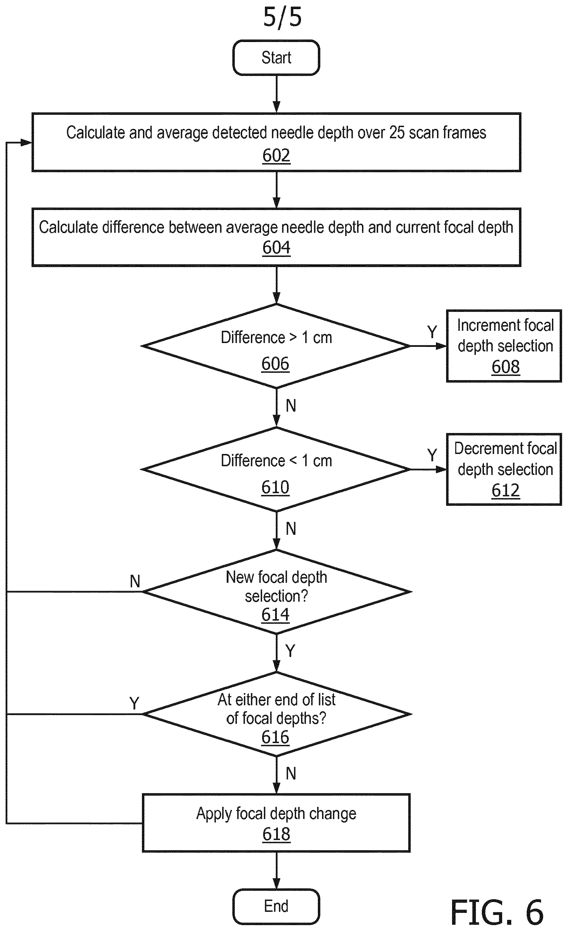

Referring to FIG. 6, a method for automatically selecting a transmit focal depth of an ultrasound probe to closely match and track the needle tip depth is illustratively shown.

In block 602, the detected needle depth is calculated and averaged over 25 scan frames.

In block 604, a difference is calculated between an average needle depth and a current focal depth.

In block 606, it is determined whether the difference is greater than a distance, e.g., 1 cm. If YES, the process flows to block 608 where the focal depth selection is incremented. If NO, the process proceeds to block 610.

In block 610, it is determined whether the difference is less than a distance, e.g., -1 cm. If YES, the process flows to block 612 where the focal depth selection is decremented. If NO, the process proceeds to block 614.

In block 614, it is determined whether there is a new focal depth selection. If NO, the process proceeds back to block 602. If YES, the process flows to block 616.

In block 616, it is determined whether the new focal depth selection is at either end of a list of focal depths. If YES, the process proceeds back to block 602. If NO, the process flows to block 618.

In block 618, a focus depth change is applied and the process flows back to block 602.

In some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

## Claims

1. An ultrasound system (210) comprising:
a probe (205) comprising a transducer array (207);
an acquisition module (211) coupled to the transducer array;
a transceiver (213) coupled to the acquisition module (211) for communicating with an adjustment module (215), wherein the adjustment module (215) is configured to automatically make adjustments to beamformed acoustic pulse characteristics of the probe (205); and
an image processor (202) for generating ultrasound images from acoustic data received by the transducer array (207);
wherein the image processor is configured to detect a position of a medical device (230) within an image field including a region of interest (242), the position being detected based on an electrical response of an acoustic sensor (234) disposed on the medical device (230) as scan lines generated by the transducer array (207) sweep across the acoustic sensor (234);
wherein the adjustments to the beamformed acoustic pulse characteristics are in accordance with the detected position of the medical device (230); and
wherein the adjustments to the beamformed acoustic pulse characteristics are selected from a set of pre-established user image adjustment selections available on a user interface.

2. The system as recited in claim 1, wherein the adjustments to the beamformed acoustic pulse characteristics are configured to optimize specificity and sensitivity of a received signal.

3. The system as recited in claim 1, wherein the adjustments to the beamformed acoustic pulse characteristics comprises a focus depth.

4. The system as recited in claim 3, wherein a relationship is established between a depth of a distal end (231) of the medical device (230) and the focus depth displayed on a display (300).

5. The system as recited in claim 3, wherein the image processor is configured to generate increments or decrements with respect to the focus depth based on a depth of a distal end of the medical device (230).

6. The system as recited in claim 3, wherein the image processor is configured to periodically compare a depth of a distal end of the medical device (230) to the focus depth.

7. The system as recited in claim 6, wherein the image processor is configured to update the focus depth after a fixed number of scan frames.

8. The system as recited in claim 3, wherein the image processor is configured to adjust the focus depth when the depth change of a medical device (230) exceeds a predetermined threshold distance.

9. The system as recited in claim 8, wherein the processor is configured to use hysteresis to prevent oscillation in a focus depth.

10. A method for determining a depth of a medical device (230) within a subject (240), the method comprising:
acquiring images of a region of interest by a probe (205) comprising a transducer array (207);
automatically making adjustments to beamformed acoustic pulse characteristics of the probe (205); and
detecting a position of a medical device (230) within an image field including a region of interest (242), the position being detected based on an electrical response of an acoustic sensor (234) disposed on the medical device (230) as scan lines generated by the transducer array (207) sweep across the acoustic sensor (234);
wherein the adjustments to the beamformed acoustic pulse characteristics are in accordance with the detected position of the medical device (230); and
wherein the adjustments to the beamformed acoustic pulse characteristics are selected from a set of pre-established user image adjustment selections available on a user interface.

11. The method as recited in claim 10, wherein the adjustments to the beamformed acoustic pulse characteristics comprises a focus depth.

12. The method as recited in claim 11, further comprising establishing a relationship between a depth of a distal end of the medical device (230) and the focus depth displayed on a display.

13. The method as recited in claim 11, wherein a depth of a distal end of the medical device (230) is used to generate increments or decrements with respect to the focus depth.

14. The method as recited in claim 11, further comprising periodically comparing a depth of a distal end of the medical device (230) to the focus depth.

15. The method as recited in claim 11, further comprising adjusting the focus depth when a depth change of the medical device (230) exceeds a predetermined threshold distance.

## Patentansprüche

1. Ultraschallsystem (210), umfassend:
eine Sonde (205), die ein Messwandler-Array (207) umfasst;
ein Aufnahmemodul (211), das mit dem Messwandler-Array gekoppelt ist;
einen Sender-Empfänger (213), der mit dem Aufnahmemodul (211) gekoppelt ist, um mit einem Anpassungsmodul (215) zu kommunizieren, wobei das Anpassungsmodul (215) dafür ausgelegt ist, automatisch Anpassungen an Eigenschaften der Sonde (205) im Hinblick auf strahlgeformte Schallimpulse vorzunehmen; und
einen Bildprozessor (202) zum Erzeugen von Ultraschallbildern aus akustischen Daten, die von dem Messwandler-Array (207) empfangen werden;
wobei der Bildprozessor dafür ausgelegt ist, eine Position einer medizinischen Vorrichtung (230) innerhalb eines Bildfelds, das eine Region von Interesse (242) einschließt, zu erfassen, wobei die Position auf Basis einer elektrischen Antwort eines an der medizinischen Vorrichtung (230) angeordneten akustischen Sensors (234) erfasst wird, wenn Abtastlinien, die von dem Messwandler-Array (207) erzeugt werden, über den akustischen Sensor (234) streichen,
wobei die Anpassungen an den Eigenschaften im Hinblick auf strahlgeformte Schallimpulse der erfassten Position der medizinischen Vorrichtung (230) entsprechen; und
wobei die Anpassungen an den Eigenschaften im Hinblick auf strahlgeformte Schallimpulse ausgewählt sind aus einem Satz von vorab festgelegten Benutzer-Bildanpassungswahlmöglichkeiten, die auf einer Benutzer-Schnittstelle verfügbar sind.

2. System nach Anspruch 1, wobei die Anpassungen an den Eigenschaften im Hinblick auf strahlgeformte Schallimpulse dafür ausgelegt sind, eine Spezifität und Sensitivität eines empfangenen Signals zu optimieren.

3. System nach Anspruch 1, wobei die Anpassungen an den Eigenschaften im Hinblick auf strahlgeformte Schallimpulse eine Fokustiefe umfassen.

4. System nach Anspruch 3, wobei eine Beziehung besteht zwischen einer Tiefe eines distalen Endes (231) der medizinischen Vorrichtung (230) und der Fokustiefe, die auf einer Anzeige (300) angezeigt wird.

5. System nach Anspruch 3, wobei der Bildprozessor dafür ausgelegt ist, Inkremente oder Dekremente in Bezug auf die Fokustiefe auf Basis einer Tiefe eines distalen Endes der medizinischen Vorrichtung (230) zu erzeugen.

6. System nach Anspruch 3, wobei der Bildprozessor dafür ausgelegt ist, periodisch eine Tiefe eines distalen Endes der medizinischen Vorrichtung (230) mit der Fokustiefe zu vergleichen.

7. System nach Anspruch 6, wobei der Bildprozessor dafür ausgelegt ist, die Fokustiefe nach einer festgelegten Anzahl von Scan-Frames zu aktualisieren.

8. System nach Anspruch 3, wobei der Bildprozessor dafür ausgelegt ist, die Fokustiefe anzupassen, wenn die Tiefenänderung einer medizinischen Vorrichtung (230) eine vorgegebene Schwellenstrecke überschreitet.

9. System nach Anspruch 8, wobei der Prozessor dafür ausgelegt ist, eine Hysterese anzuwenden, um eine Schwankung einer Fokustiefe zu verhindern.

10. Verfahren zum Bestimmen einer Tiefe einer medizinischen Vorrichtung (230) innerhalb eines Subjekts (240), wobei das Verfahren umfasst:
Aufnehmen von Bildern einer Region von Interesse durch eine Sonde (205), die ein Messwandler-Array (207) umfasst;
automatisch Vornehmen von Anpassungen an Eigenschaften der Sonde (205) im Hinblick auf strahlgeformte Schallimpulse; und
Erfassen einer Position einer medizinischen Vorrichtung (230) innerhalb eines Bildfelds, das eine Region von Interesse (242) einschließt, wobei die Position auf Basis einer elektrischen Antwort eines an der medizinischen Vorrichtung (230) angeordneten akustischen Sensors (234) erfasst wird, wenn Abtastlinien, die von dem Messwandler-Array (207) erzeugt werden, über den akustischen Sensor (234) streichen,
wobei die Anpassungen an den Eigenschaften im Hinblick auf strahlgeformte Schallimpulse der erfassten Position der medizinischen Vorrichtung (230) entsprechen; und
wobei die Anpassungen an den Eigenschaften im Hinblick auf strahlgeformte Schallimpulse ausgewählt sind aus einem Satz von vorab festgelegten Benutzer-Bildanpassungswahlmöglichkeiten, die auf einer Benutzer-Schnittstelle verfügbar sind.

11. Verfahren nach Anspruch 10, wobei die Anpassungen an den Eigenschaften im Hinblick auf strahlgeformte Schallimpulse eine Fokustiefe umfassen.

12. Verfahren nach Anspruch 11, ferner umfassend das Einrichten einer Beziehung zwischen einer Tiefe eines distalen Endes der medizinischen Vorrichtung (230) und der Fokustiefe, die auf einer Anzeige angezeigt wird.

13. Verfahren nach Anspruch 11, wobei eine Tiefe eines distalen Endes der medizinischen Vorrichtung (230) verwendet wird, um Inkremente oder Dekremente in Bezug auf die Fokustiefe zu erzeugen.

14. Verfahren nach Anspruch 11, ferner das periodische Vergleichen einer Tiefe eines distalen Endes der medizinischen Vorrichtung (230) mit der Fokustiefe umfassend.

15. Verfahren nach Anspruch 11 ferner das Anpassen der Fokustiefe umfassend, wenn eine Tiefenänderung einer medizinischen Vorrichtung (230) eine vorgegebene Schwellenstrecke überschreitet.

## Revendications

1. Système à ultrasons (210) comprenant:
une sonde (205) comprenant un réseau de transducteurs (207) ;
un module d'acquisition (211) couplé au réseau de transducteurs;
un émetteur-récepteur (213) couplé au module d'acquisition (211) pour communiquer avec un module de réglage (215), dans lequel le module de réglage (215) est configuré pour effectuer automatiquement des réglages aux caractéristiques d'impulsions acoustiques en forme de faisceau de la sonde (205); et
un processeur d'image (202) pour générer des images ultrasonores à partir de données acoustiques reçues par le réseau de transducteurs (207);
dans lequel le processeur d'image est configuré pour détecter une position d'un dispositif médical (230) dans un champ d'image comprenant une zone d'intérêt (242), la position étant détectée en fonction d'une réponse électrique d'un capteur acoustique (234) disposé sur le dispositif médical un dispositif (230) sous la forme de lignes de balayage générées par le réseau de transducteurs (207) à un balayage du capteur acoustique (234);
dans lequel les réglages des caractéristiques d'impulsions acoustiques en forme de faisceau sont en accord avec la position détectée du dispositif médical (230); et
dans lequel les réglages des caractéristiques d'impulsions acoustiques en forme de faisceau sont sélectionnés à partir d'un ensemble de sélections de réglage d'image utilisateur préétablies disponibles sur une interface utilisateur.

2. Système selon la revendication 1, dans lequel les réglages des caractéristiques d'impulsions acoustiques en forme de faisceau sont configurés pour optimiser la spécificité et la sensibilité d'un signal reçu.

3. Système selon la revendication 1, dans lequel les réglages des caractéristiques d'impulsions acoustiques en forme de faisceau comprennent une profondeur de focalisation.

4. Système selon la revendication 3, dans lequel une relation est établie entre une profondeur d'une extrémité distale (231) du dispositif médical (230) et la profondeur de mise au point affichée sur un écran (300) .

5. Système selon la revendication 3, dans lequel le processeur d'image est configuré pour générer des incréments ou des décréments par rapport à la profondeur de mise au point en fonction d'une profondeur d'une extrémité distale du dispositif médical (230).

6. Système selon la revendication 3, dans lequel le processeur d'image est configuré pour comparer périodiquement une profondeur d'une extrémité distale du dispositif médical (230) à la profondeur de mise au point.

7. Système selon la revendication 6, dans lequel le processeur d'image est configuré pour mettre à jour la profondeur de mise au point après un nombre fixe de trames de balayage.

8. Système selon la revendication 3, dans lequel le processeur d'image est configuré pour ajuster la profondeur de mise au point lorsque le changement de profondeur d'un dispositif médical (230) est supérieur à une distance seuil prédéterminée.

9. Système selon la revendication 8, dans lequel le processeur est configuré pour utiliser l'hystérésis pour empêcher une oscillation dans une profondeur de focalisation.

10. Procédé de détermination d'une profondeur d'un dispositif médical (230) à l'intérieur d'un sujet (240), ledit procédé comprenant:
l'acquisition des images d'une zone d'intérêt par une sonde (205) comprenant un réseau de transducteurs (207) ;
la réalisation automatique des réglages aux caractéristiques d'impulsions acoustiques en forme de faisceau de la sonde (205); et
la détection d'une position d'un dispositif médical (230) dans un champ d'image comprenant une zone d'intérêt (242), la position étant détectée en fonction d'une réponse électrique d'un capteur acoustique (234) disposé sur le dispositif médical (230) sous la forme de lignes de balayage généré par le réseau de transducteurs (207) à un balayage du capteur acoustique (234);
dans lequel les réglages des caractéristiques d'impulsions acoustiques en forme de faisceau sont en accord avec la position détectée du dispositif médical (230); et
dans lequel les réglages des caractéristiques d'impulsions acoustiques en forme de faisceau sont sélectionnés à partir d'un ensemble de sélections de réglage d'image utilisateur préétablies disponibles sur une interface utilisateur.

11. Procédé selon la revendication 10, dans lequel les réglages des caractéristiques d'impulsions acoustiques en forme de faisceau comprennent une profondeur de focalisation.

12. Procédé selon la revendication 11, comprenant en outre l'établissement d'une relation entre une profondeur d'une extrémité distale du dispositif médical (230) et la profondeur de mise au point affichée sur un écran.

13. Procédé selon la revendication 11, dans lequel une profondeur d'une extrémité distale du dispositif médical (230) est utilisée pour générer des incréments ou des décréments par rapport à la profondeur de mise au point.

14. Procédé selon la revendication 11, comprenant en outre la comparaison périodique d'une profondeur d'une extrémité distale du dispositif médical (230) avec la profondeur de mise au point.

15. Procédé selon la revendication 11, comprenant en outre le réglage de la profondeur de mise au point lorsqu'un changement de profondeur du dispositif médical (230) est supérieur à une distance seuil prédéterminée.
